**Europäisches Patentamt**

**European Patent Office**

**Office européen des brevets**

(19)

(11) Numéro de publication: **0 022 046**
**B1**

(12) # FASCICULE DE BREVET EUROPEEN

(45) Date de publication du fascicule du brevet: **25.08.82**  (51) Int. Cl.³: **A 61 K 35/78**

(21) Numéro de dépôt: **80420074.9**

(22) Date de dépôt: **20.06.80**

---

(54) Nouvelle composition médicamenteuse à base de plantes et procédé de préparation.

---

(30) Priorité: **25.06.79 FR 7917258**

(43) Date de publication de la demande:
**07.01.81 Bulletin 81/1**

(45) Mention de la délivrance du brevet:
**25.08.82 Bulletin 82/34**

(84) Etats contractants désignés:
**BE CH DE GB IT LI LU NL SE**

(56) Documents cités:
**FR - A - 1 433 383**
**FR - M - 4 209**
**FR - M - 5 975**

Le dossier contient des informations techniques
présentées postérieurement au dépôt de la
demande et ne figurant pas dans le présent
fascicule.

(73) Titulaire: **ETABLISSEMENT RINRONE (Société de droit du Liechtenstein)**
**Mauren (LI)**

(72) Inventeur: **Lloppart, Jean-Paul**
**17, avenue de la Mer**
**F-66200 Alenya (FR)**

(74) Mandataire: **Laurent, Michel et al,**
**Bureaux Chalin A1 20, rue Louis Chirpaz Boîte Postale 32**
**F-69130 Lyon-Ecully (FR)**

---

Courier Press, Leamington Spa, England.

## Nouvelle composition médicamenteuse à base de plantes et procédé de préparation

L'invention concerne un nouveau médicament à usage externe, à base de plantes, destiné notamment au traitement des plaies.

Pour le traitement des plaies, on a depuis longtemps suggéré d'utiliser des solutions de produits chimiques, telles que le mercurochrome ou les ammoniums quaternaires. Bien que largement utilisées, notamment lors des premières interventions, ces solutions présentent l'inconvénient de rendre fragile le terrain cellulaire où se trouve la blessure et d'avoir une efficacité partielle.

On a également largement utilisé dans le même but des antibiotiques ou des sulfamides. Bien que ces produits présentent de nombreux avantages indiscutables, ils ont parfois l'inconvénient, outre une certaine toxicité et une action irritative au point d'application, pour certains d'entre eux, de provoquer l'apparition de souche résistante aux bactéries.

L'invention pallie ces inconvénients. Elle concerne une composition médicamenteuse à usate externe, à base de plantes, facile à mettre en oeuvre et présentant l'avantage de reconstituer le terrain cellulaire.

Cette composition liquide de base se caractérise en ce qu'elle contient:
— de 450 à 1000 ml de teinture mère de Centella Asiatica,
— de 11 à 65 ml d'huile essentielle naturelle de Lavandula Officinalis,
— de 5 à 10 ml d'huile essentielle naturelle de Thymus Officinalis,
— de 5 à 20 ml d'huile essentielle naturelle de Rosmarinus Officinalis,
— de 4 à 14 ml de teinture mère de Aesculus Hippocastunum.

Avantageusement, cette composition peut contenir en outre le ou les constituants suivants:
— de 0,1 à 6 ml de teinture mère de Medicago Sativa,
— de 0,1 à 6 ml de teinture mère de Carlina Acaulis,
— de 2 à 15 ml d'huile essentielle naturelle de Capressus Officinalis.

Les désignations ci-dessus sont celles du Codex, c'est-à-dire du dictionnaire médical officiel en France (dénommé aussi "Pharmacopée").

Comme on le sait, une "huile essentielle" est une huile, généralement volatile, obtenue par distillation de substances naturelles aromatiques d'origine végérale. Ces huiles sont largement utilisées en pharmacie et en parfumerie.

En revanche, une "teinture mère" résulte de la macération en milieu alcoolique pur, pendant une durée pratique de l'ordre de plusieurs jours, de plantes. Ces teintures mères, également largement utilisées en pharmacie, se présentent donc à une concentration maximum.

La "Centella Asiatica" est une plante de la famille des ombellifères, dénommée aussi parfois Hydrocotyle, bien connue pour son action de régénération contrôlée cytophilactique. De préférence, ce macérat est effectué dans de l'alcool à au moins 80°C et ce, pendant une durée appréciable, plus importante que les durées usuelles de macération des teintures actuelles d'Hydrocotyle du Codex.

L'emploi de cette plante comme médicament cicatrisant à usage externe est connu depuis fort longtemps (voir parexample brevet français spécial de médicament BSM 4209 M). Son principal principe actif thérapeutique est l'extrait triterpénique.

La "Lavandula", dénommée de manière connue lavande est une plante de la famille des labiées. L'huile essentielle de lavande a pour principaux constituants des éthers de linalyle et de géranyle, géranio linabol, cinéol, d-bornéol, limonène, 1-pinène, caryophilène, éthers butyrique et valérianique, coumarine, des carbures terpéniques des alcools terpéniques et surtout des esters de linalol (acetate) et du géraniol. Cette huile essentielle est connue pour son action à usage externe antiseptique et cicatrisante.

La "Thymus", dénommé couramment thym, est un plante également de la famille des labiées et dont l'huile essentielle a pour principaux constituants des essences (phénols) de thymol, de carvacrol, de cymol, de bornéol, de linalol et du tanin, du paracymène et des dérivés terpéniques, des terpinénes et un peu de cynéol. Cette huile est bien connue pour son action à usage externe antiseptique, bactéricide et antiputride.

Le "Rosmarinus", dénommé couramment romarin, est aussi une plante de la famille des labiées, dont l'huile essentielle a pour principaux constituants des huiles essentielles de pinène, de camphène, de cinéol, de bornéol, des camphres, de l'acétate de bornyle. Cette huile a une action externe résolutive antiseptique et cicatrisante.

L'"Aesculus Hippocastunum", dénommé couramment le maron d'Indes, est une plante de la famille des hippocastanacées dont la teinture mère, formée à partir de la graine et de son tégument contient comme principes actifs essentiels des saponides triterpéniques, dont notamment l'aescine, et des dérivés flavoniques de la quercétine et du Kampférol et des tanins catéchiques et a pour principaux constituants des saponines, des tanins, un glucoside, des flavones. Cette teinture est bien connue comme activateur sanguin et pour ses propriétés anti-inflammatoires.

La "Medicago Sativa", dénommée couramment luzerne, est une plante de la famille des légumineux, dont la teinture mère a pour principaux constituants une provitamine A (bétacarotène), des vitamines C, D et $K_1$ et des sels minéraux de calcium, de potassium, de fer et de phosphore. Cette teinture a une action recalcifiante et antihémorragique.

La "Carlina Acaulis", dénommée couramment carline, est une plante de la famille des composées dont la teinture mère a pour principaux constituants des huiles essentielles: dérivés acétyléniques (oxyde de carline : furyl — benzylacétylène). Cette teinture a une action reconstituante, antidermatosique, tonique, antibiotique et sert d'activateur du sympathique.

Le "Cupressus" est une plante de la famille des conifères. L'huile essentielle de Cupressus, dénommé couramment cyprès, a pour constituants essentiels des tanins, des huiles essentielles: d-pinène, d-campène, d-sylvestène, cymène, une cétone, du sabinol, un alcool terpénique, de l'acide valérianique et du camphre de cyprès. Cette huile essentielle a une action vasculaire phlébotonique directe, c'est-à-dire de vaso-constricteur et de tonifiant des veines.

Chacune des huiles essentielles et des teintures mères ci-dessus sont déjà bien connues pour leurs applications pharmaceutiques. La composition selon l'invention consiste à assembler dans des proportions et sous des formes déterminées et précises ces constituants, ce qui provoque une synergie et une inter-réaction entre eux. La composition finale de base obtenue présente des effets et des résultats nettement améliorés par rapport à ce que l'on obtenait avec chaque constituant pris isolément et successivement ou de manière cumulée, ce qui est inattendu et surprenant.

On a déterminé que l'on obtient les meilleurs résultats avec une composition de base contenant:
— 986 ml de teinture mère d'hydrocotyle,
— 65 ml d'huile essentielle de lavande,
— 10 ml d'huile essentielle de thym,
— 17 ml d'huile essentielle de romarin,
— 14 ml de teinture mère de marron d'Indes, et avantageusement:
— 1,7 ml de teinture mère de luzerne,
— 1,5 ml de teinture mère de carline,
— 9 ml d'huile essentielle de cyprès.

L'addition de ces trois derniers constituants améliorant l'activité des principes actifs, notamment par suite de l'orientation spécifique de chacun d'eux.

Cette composition de base se présente sous forme d'une teinture liquide de couleur vert foncé. Le plus généralement, elle peut être utilisée telle que, c'est-à-dire à concentration maximum. Toutefois, pour certaines applications ne nécessitant pas d'activité optimum, on peut diluer cette composition par exemple dans l'alcool. On peut par exemple diluer un litre de cette composition de base dans 300 cm³ d'éthanol.

Pour fabriquer cette composition, on prépare de manière connue les huiles essentielles ou les teintures mères de chacun des constituants. A température ambiante, on mélange séparément sous agitation toutes les teintures et toutes les huiles essentielles et enfin, toujours à température ambiante, on mélange sous agitation l'ensemble en incorporant les huiles essentielles dans les teintures.

En pratique, les compositions liquides pour le traitement des plaies conformes à l'invention peuvent être utilisées de manière connue, par exemple par application sur des compresses. Avantageusement, on utilise toutefois cette composition par pulvérisation directe sur la plaie.

Les propriétés pharmacologiques de ces compositions sont essentiellement d'être cicatrisante et antiseptique.

Par ailleurs, ces compositions ont été testées sur le plan de la toxicité aigüe, par voie orale chez le rat et la souris, et les doses léthales 50% s'établissent ainsi:

| — *rat* | : | mâles | 11,41 g |
|---------|---|-------|---------|
| | : | femelles | 11,59 g |
| — *souris* | : | mâles | 11,29 g |
| | : | femelles | 11,89 g |

On peut donc en déduire que ces compositions sont très peu toxiques.

A titre d'application thérapeutique, pour l'animal ou l'humain, on peut citer la cicatrisation et la désinfection de:
— plaies ouvertes d'origines diverses,
— plaies atones d'origines diverses,
— ulcères variqueux,
— escarres,
— mycoses,
— dermatoses.

On peut également citer les plaies de toute nature par blessure ou brûlure, les escarres et les ulcères de jambe, survenant soit chez les personnes atteintes antérieurement de phlébite, soit chez les variqueux. En ce qui concerne les plaies par blessures, l'expérimentation a démontré que la vitesse de cicatrisation des plaies traitées par les compositions selon l'invention était pratiquement le double de celles traitées par d'autres procédés, notamment avec des antibiotiques ou autres medications classiques.

A titre indicatif, on a déterminé que la posologie moyenne était de 1 à 6 cm³ par jour, étant entendu que cette posologie peut être augmentée s'il y a une résistivité.

La manière dont l'invention peut être réalisée et les avantages qui en découlent ressortiront mieux des exemples de réalisation qui suivent donnés à titre indicatif et non limitatif.

Exemple 1

D'une manière connue, on prépare selon le cas soit une teinture mère, soit une huile essentielle de chacun des constituants de la composition de base dans de l'alcool.

A température ambiante (20°C), on mélange dans l'ordre sous agitation:
— 986 ml de teinture mère de Centella Asiatica (Hydrocotyle),
— 14 ml de teinture mère de Aescullus Hippocastunum (marron d'Inde),
— 1,5 ml de teinture mère de Carlina Acaulis (carli-25 ne),
— 1,7 ml de teinture mère de Medicago Sativa (luzerne).

Indépendamment et toujours à température ambiante (20°C), on mélange dans l'ordre également sous agitation:
— 65 ml d'huile essentielle naturelle de Lavandula Officinalis (lavande),
— 17 ml d'huile essentielle naturelle de Rosmarinus Officinalis (rosmarin),
— 10 ml d'huile essentielle naturelle de Thymus Officinalis (thym),
— 9 ml d'huile essentielle naturelle de Cupressus Officinalis (cyprès).

On mélange ensuite, toujours à température ambiante et sous agitation chacun des mélanges de teintures mères et d'huiles essentielles en versant le mélange d'huiles dans le mélange de teintures.

On obtientuune composition liquide de 1104,2 ml de couleur vert foncé alcoolique de 92°.

A l'aide d'un pulvérisateur (pulvérisation moyenne d'environ 1 cm³), on projette cette composition sur un ulcère variqueux externe avec atonie d'un sujet humain normal à raison de 4 à 6 pulvérisations par jour.

Au bout de quatorze jours, la plaie se referme et celle-ci est parfaitement guérie au bout de quarante jours de traitement.

A titre comparatif, les traitements actuels par antibiotiques, sulfamides ou corticoides durent souvent plusieurs mois et donnent des résultats parfois aléatoires.

### Exemple 2

A l'aide de la composition de l'exemple 1, on traite une plaie ouverte, provoquée par blessure dans la chair, d'un échantillon de rats sains calibrés et ce, à raison de 3 à 4 pulvérisations par jour.

Selon l'importance des plaies (largeur, profondeur), celles-ci se sont refermées au bout de deux à quatre jours, ce qui diminue considérablement le risque d'infection.

En revanche, à titre de comparaison, le traitement des nêmes blessures sur un échantillon comparable des mêmes sujets, aurait nécessité des piqûres d'antibiotiques et aurait présenté une évolution cellulaire ralentie, ce qui bien souvent entraîne la putréfaction des chairs.

### Exemple 3

A l'aide de la même composition qu'à l'exemple 1, on traite, sur un sujet humain grabataire, un escarre à raison de six pulvérisations par jour.

Après quinze jours de traitement, on constate que les éléments putrides ont disparu et après vingt jours, l'escarre peut être considéré comme guéri.

Cela est d'autant plus inattendu que les méthodes utilisées actuellement ne permettent pas de traiter convenablement ce genre de plaies qui pratiquement se referment très difficilement.

### Exemple 4

On utilise la composition de l'exemple 1 à des concentrations différentes dans l'éthanol à 90° pour traiter différentes plaies à raison de deux à cinq pulvérisations par jour. On a regroupé les résultats dans le tableau de la page 9 ci-après.

Comme déjà dit, l'action cicatrisante de la Centella Asiatica était connue depuis fort longtemps. Toutefois, cette action est trop souvent rapide, de sorte qu'avec des plaies profondes, on peut parfois cicatriser en surface, alors que le foyer d'infection subsiste encore à l'intérieur. En revanche, la composition selon l'invention, grâce à la synergie de ce composé majoritaire pondéralement avec les autres composés, conduit à une action cicatrisante contrôlée avec régénération cellulaire, sans foyer infectieux sous-cutané.

Ainsi, on a déterminé sur des plaies effectuées sur des lots de cinq rats et infectés par un staphylocoque doré que la cicatrisation était complète, c'est-à-dire l'épidermisation totale sans laisser aucune trace:
— au bout de cinquante et un (51) jours pour l'ensemble du lot avec la composition selon exemple 1 et ce, à raison de deux pulvérisations d'une goutte par jour,
— contre soixante dix (70) jours pour un lot identique témoin,
— contre soixante (60) jours pour un lot identique traité avec une composition de teinture mère de Centella Asiatica (hydrocotyle),
— contre soixante cinq (65) jours pour un lot identique traité avec une composition contenant 14 ml de teinture mère de marron d'inde, 1,5 ml de teinture mère de carline et 1,7 ml de teinture mère de luzerne,
— contre soixante seize (76) jours pour un lot identique traité avec un mélange d'hydrocotyle et d'une composition contenant 65 ml d'huile essentielle de lavande, 17 ml d'huile essentielle de romarin, 10 ml d'huile essentielle de thym et 9 ml d'huile essentielle de cyprès additionnée d'hydrocotyle.

En d'autres termes, la combinaison des différents constituants permet tout à la fois d'avoir une meilleure action cicatrisante, de lutter contre l'infection et l'inflammation sans nuire aux autres propriétés. Ainsi, la synergie des différents constituants (teintures, huiles essentielles) non seulement présente les avantages connus pour chacun de ces constituants, mais surtout améloire et renforce l'action propre de chaque constituant, sans nuire aux autres propriétés, ce qui démontre l'inter-action et était totalement inattendu.

| Dosage de la composition dans de l'alcool 90° | Blessure cutanée | Brûlure 2° degré | Plaie atone | Ulcère variqueux | Escarre |
|---|---|---|---|---|---|
| 3% | Se cicatrise au bout de 8 jours | N'a pas tendance à s'infecter | N'a pas tendance à s'infecter | NUL | NUL |
| 7% | Se cicatrise au bout de 7 jours | IDEM | IDEM | NUL | NUL |
| 15% | 6 jours | Régénération cellulaire lente | Début de cicatrisation | Couche purulente d'un gris-vert, commence à se détacher | Désodorisation plus accentuée |
| 25% | 5 jours | Délimite rapidement les tissus sains et malades | Cicatrisation graduelle, action désodorisante | Apparition de bourgeons | IDEM |
| 50% | 5 jours | Régénération rapide | La plaie se comble | La plaie a un très bel aspect | Délimitation nette des bords nécrosés |
| 75% | 4 jours | Cicatrisée | Plaie presque fermée | Fond bourgeonnant | Régénération progressive, les chairs se reforment |
| 100% | 48 Heures | Cicatrisée au bout de 6 jours | Cicatrisée | Plaie presque fermée | En très bonne voie de cicatrisation |

## Revendications

1. Composition liquide médicamenteuse à base de plantes, notamment de Centella Asiatica, à usage externe pour le traitement des plaies, caractérisée en ce qu'elle contient:
— de 450 à 1000 ml de teinture mère de Centella Asiatica,
— de 11 à 65 ml d'huile essentielle naturelle de Lavandula Officinalis,
— de 5 à 10 ml d'huile essentielle naturelle de thymus officinalis,
— de 5 à 20 ml d'huile essentielle naturelle de Rosmarinus Officinalis,
— de 4 à 14 ml de teinture mère de Aesculus Hippocastunum.

2. Composition selon revendication 1, caractérisée en ce qu'elle contient en outre:
— de 0,1 à 6 ml de teinture mère de Medicago Sativa,
— de 0,1 à 6 ml de teinture mère de Carlina Acaulis,
— de 2 à 15 ml d'huile essentielle naturelle de Cupressus Officinalis.

3. Composition selon l'une des revendications 1 et 2, caractérisée en ce qu'elle est diluée dans de l'éthanol.

4. Composition liquide médicamenteuse à usage externe pour le traitement des plaies, selon la revendication 1, caractérisée en ce qu'elle contient:
— 986 ml de teinture mère de Centella Asiatica,
— 65 ml d'huile essentielle de Lavandula Officinalis,
— 10 ml d'huile essentielle de Thymus Officinalis,
— 17 ml d'huile essentielle de Rosmarinus Officinalis,
— 14 ml de teinture mère de Aesculus Hippocastunum.

5. Composition selon revendication 4, caractérisée en ce qu'elle contient également dilués dans de l'éthanol:
— 1,7 ml de teinture mère de Medicago Sativa,
— 1,5 ml de teinture mère de Carlina Acaulis,
— 9 ml d'une huile essentielle de Cupressus Officinalis.

6. Procédé pour la préparation d'une composition liquide médicamenteuse à usage externe pour le traitement des plaies, selon l'une des revendications 1 à 5, caractérisé en ce qu'il consiste:
— tout d'abord, à mélanger sous agitation:
— de 450 à 1000 ml d'une teinture mère de Centella Asiatica et de 4 à 14 ml d'une teinture mère de Aesculus Hippocastunum,
— puis, parallélement et séparément, à mélanger également sous agitation:
— de 11 à 65 ml d'huile essentielle de Lavandula Officinalis,
— de 5 à 10 ml d'huile essentielle de Thymus Officinalis,
— et de 5 à 20 ml d'huile essentielle de Rosmarinus Officinalis,
— et enfin, à mélanger sous agitation les mélanges précédemment obtenus en versant les huiles essentielles dans les teintures mères.

7. Procédé selon la revendication 6, caractèrisé en ce que:
— aux teintures mères, on ajoute de 0,1 à 6 ml de teinture mère de Carlina Acaulis et de 0,1 à 6 ml de teinture mère de Medicago Sativa;
— aux huiles essentielles, de 2 à 15 ml d'une huile essentielle de Cupressus Officinalis.

8. Procédé selon la revendication 7, caractérisée en ce qu'on mélange à température ambiante:
— 986 ml de teinture mère de Centella Asiatica,
— 14 ml de teinture mère de Aesculus Hippocastunum,
— 1,7 ml de teinture mère de Medicago Sativa,
— 1,5 ml de teinture mère de Carlina Acaulis,
— 65 ml d'huile essentielle naturelle de Lavandula Officinalis,
— 10 ml d'huile essentielle naturelle de Thymus Officinalis,
— 17 ml d'huile essentielle naturelle de Rosmarinus Officinalis,
— 9 ml d'une huile essentielle naturelle de Cupressus Officinalis.

## Patentansprüche

1. Flüssige Arzneizubereitung auf Pflanzenbasis, besonders der Centella Asiatica, zur äußerlichen Anwendung zur Behandlung von Wunden, dadurch gekennzeichnet, daß sie enthält:
— 450 bis 1000 ml Urtinktur der Centella Asiatica,
— 11 bis 65 ml natürliches aetherisches Öl der Lavandual Officinalis,
— 5 bis 10 ml natürliches aetherisches Öl der Thymus Officinalis,
— 5 bis 20 ml natürliches aetherisches Öl der Rosmarinus Officinalis,
— 4 bis 14 ml Urtinktur der Aesculus Hippocastanum.

2. Arzneizubereitung nach Anspruch 1, dadurch gekennzeichnet, daß sie außerdem enthält:
— 0,1 bis 6 ml Urtinktur der Medicago Sativa,
— 0,1 bis 6 ml Urtinktur der Carlina Acaulis,
— 2 bis 15 ml natürliches aetherisches Öl der Cubressus Officinalis.

3. Arzneizubereitung nach einem der Ansprüche 1 oder 2, dadurch gekennzeichnet, daß sie in Äthanol verdünnt wird.

4. Flüssige Arzneizubereitung zur äußerlichen Anwendung zur Wundbehandlung gemäß Anspruch 1, dadurch gekennzeichnet, daß sie enthält:
— 986 ml Urtinktur der Centella Asiatica,

— 65 ml aetherisches Öl der Lavandula Officinalis,

— 10 ml aetherisches Öl der Thymus Officinalis,

— 17 ml aetherisches Öl der Rosmarinus Officinalis,

— 14 ml aetherisches Öl der Aesculus Hippocastanum.

5. Arzneizubereitung nach Anspruch 4, dadurch gekennzeichnet, daß sie ebenfalls aufgelöst in Äthanol enthält:

— 1,7 ml Urtinktur der Medicago Sativa,

— 1,5 ml Urtinktur der Carlina Acaulis,

— 9 ml aetherisches Öl der Cupressus Officinalis.

6. Verfahren zur Herstellung einer flüssigen Arzneizubereitung zur äußerlichen Anwendung für die Wundbehandlung gemäß einem der Ansprüche 1 bis 5, dadurch gekennzeichnet, daß

— zuerst durch Schütteln

— 450 bis 1000 ml Urtinktur der Centella Asiatica und 4 bis 14 ml Urtinktur der Aesculus Hippocastanum vermischt werden,

— dann, parallel dazu und separat, ebenfalls durch Schütteln

— 11 bis 65 ml aetherisches Öl der Lavandula Officinalis,

— 5 bis 10 ml aetherisches Öl der Thymus Officinalis

— und 5 bis 20 ml aetherisches Öl der Rosmarinus Officinalis vermischt werden

— und letztlich durch Schütteln die vorgenannten Mischungen vermischt werden, indem die aetherischen Öle in die Urtinkturen gegeben werden.

7. Verfahren nach Anspruch 6, dadurch gekennzeichnet, daß

— zu den Urtinkturen 0,1 bis 6 ml Urtinktur der Carlina Acaulis und 0,1 bis 6 ml Urtinktur der Medicago Sativa und

— zu den aetherischen Ölen 2 bis 15 ml aetherisches Öl der Cupressus Officinalis hinzugegeben werden.

8. Verfahren nach Anspruch 7, dadurch gekennzeichnet, daß bei Zimmertemperatur vermischt werden:

— 986 ml Urtinktur der Centella Asiatica,

— 14 ml Urtinktur der Aesculus Hippocastanum,

— 1,7 ml Urtinktur der Medicago Sativa,

— 1,5 ml Urtinktur der Carlina Acaulis,

— 65 ml natürliches aetherisches Öl der Lavandula Officinalis,

— 10 ml natürliches aetherisches Öl der Thymus Officinalis,

— 17 ml natürliches aetherisches Öl der Rosmarinus Officinalis,

— 9 ml natürliches aetherisches Öl der Cupressus Officinalis.

## Claims

1. Liquid medicinal composition based on plants, specially Centella Asiatica, for external use for treating wounds, characterized in that it comprises:

— from 450 to 1,000 ml of base tincture of Centella Asiatica,

— from 11 to 65 ml of natural essential oil of Lavendula Officinalis,

— from 5 to 10 ml of natural essential oil of Thymus Officinalis,

— from 5 to 20 ml of natural essential oil of Rosmarinus Officinalis,

— from 4 to 14 ml of base tincture of Aesculus Hippocastunum.

2. Composition according to claim 1, characterized in that it comprises also:

— from 0,1 to 6 ml of base tincture of Medicago Sativa,

— from 0.1 to 6 ml of base tincture of Carlina Acaulis,

— from 2 to 15 ml of natural essential oil of Cupressus Officinalis.

3. Composition according to claims 1 and 2, characterized in that it is diluted in ethanol.

4. Liquid medicinal composition for external use for treating wounds according to claim 1, characterized in that it comprises:

— 986 ml of base tincture of Centella Asiatica,

— 65 ml of essential oil of Lavandula Officinalis,

— 10 ml of essential oil of Thymus Officinalis,

— 17 ml of essential oil of Rosmarinus Officinalis,

— 14 ml of base tincture of Aesculus Hippocastunum.

5. Composition according to claim 4, characterized in that it comprises also diluted into ethanol:

— 1.7 ml of base tincture of Medicago Sativa,

— 1.5 ml of base tincture of Carlina Acaulis,

— 9 ml of essential oil of Cupressus Officinalis.

6. Process for the preparation of a liquid medicinal composition for external use for treating wounds according to claims 1 to 5, characterized in that it consists:

— first, to mix whilst stirring:

— from 450 to 1000 ml of base tincture of Centella Asiatica and from 4 to 14 ml of base tincture of Aesculus Hippocastunum,

— then, independently and separately, to mix also whilst stirring:

— from 11 to 65 ml of essential oil of Lavandula Officinalis,

— from 5 to 10 ml of essential oil of Thymus Officinalis,

— and from 5 to 20 ml of essential oil of Rosmarinus Officinalis,

— and, finally to mix whilst stirring the above mixtures, the essentials oils being poured into the base tinctures.

7. Process according to claim 6, characterized by the fact that it is added:

— to base tinctures from 0,1 to 6 ml of base tincture of Carlina Acaulis and from 0,1 to 6 ml of base tincture of Medicago Sativa;

— to essential oils from 2 to 15 ml of essential oil of Cupressus Officinalis.

8. Process according to claim 7, characterized in that it mixes at ambient temperature:

— 986 ml of base tincture of Centella Asiatica,

— 14 ml of base tincture of Aesculus Hippocastunum,

— 1.7 ml of base tincture of Medicago Sativa,

— 1.5 ml of base tincture of Carlina Acaulis,

— 65 ml of natural essential oil of Lavandula Officinalis,

— 10 ml of natural essential oil of Thymus Officinalis,

— 17 ml of natural essential oil of Rosmarinus Officinalis,

— 9 ml of natural essential oil of Cupressus.